(19) Europäisches Patentamt
European Patent Office
Office européen des brevets

(11) Publication number: 0 054 430
B1

(12) EUROPEAN PATENT SPECIFICATION

(45) Date of publication of patent specification: 13.03.85

(51) Int. Cl.$^4$: C 07 C 91/04, C 07 C 93/04, C 08 G 18/32, C 08 G 18/50, C 08 G 65/28

(21) Application number: 81305872.4

(22) Date of filing: 14.12.81

# (54) Tertiary amino polyols.

(30) Priority: 15.12.80 US 216212

(43) Date of publication of application:
23.06.82 Bulletin 82/25

(45) Publication of the grant of the patent:
13.03.85 Bulletin 85/11

(84) Designated Contracting States:
BE DE FR IT NL SE

(56) References cited:
DE-A-2 331 290
DE-A-2 424 636
DE-A-2 707 659

(73) Proprietor: HENKEL CORPORATION
7900 West 78th Street
Minneapolis Minnesota 55435 (US)

(72) Inventor: Rogier, Edgar Robert
16400 Hidden Valley Road
Minnetonka Minnesota 55343 (US)

(74) Representative: Watkins, Arnold Jack et al
European Patent Attorney Frank B. Dehn & Co.
Imperial House 15-19 Kingsway
London WC2B 6UZ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 054 430 B1

Courier Press, Leamington Spa, England.

## Description

The present invention relates to compounds which contain a plurality of hydroxyl groups and tertiary amine functionality.

It has recently been determined that high molecular weight alcohols may be prepared which are liquid in nature. Such materials are described in our United States Patent 4,216,343 dated August 5, 1980. It has been determined that certain derivatives leading to the starting material of such alcohols may be valuably converted to amino alcohols which may thereafter be derivatized to amino alcohols and thereafter to an alkylene oxide adduct of the amino alcohol. Such materials are particularly valuable in that they contain both hydroxyl groups and tertiary amine functionality. The tertiary amine functionality is valuable in that it provides an autocatalytic reactant for urethane formation.

The possibility of autocatalysis in urethane formation is envisaged by European Patent Application Publication No. 12927A, which discusses the use of certain poly(oxyalkylene) adducts of di- or polyamines as autocatalytic reagents in the preparation of polyurethane binders for foundry sands.

Poly(oxyalkylene) adducts of amines as reagents of modifiers in urethane formation are also discussed in European Patent Applications Publication Nos. 9755A, 17947A and 17948A.

Certain work has also been done by R. Lai and is presented in an article entitled *Obtention de Derives Bio Fonctionnels* at Rev. Fr. Corps. Gras. *17*:455 (1970). Therein, certain derivatives of alcohols are suggested.

The amino polyols suggested by the published European Patent Applications referred to above are relatively hydrophilic or hydroscopic and we have found that by the inclusion in a tertiary amino polyol of a relatively long branched aliphatic chain having a single terminal hydroxyl functionality, tertiary amino polyols can be produced which are capable of acting as autocatalytic reagents in the formation of urethane products of reduced water sensitivity and which are particularly suitable for the production of urethane coatings and elastomers and for reaction injection moulding compositions.

According to one aspect of the present invention we provide a tertiary amino polyol of formula A

$$CH_3(CH_2)_xCH[(CH_2)_yOH](CH_2)_zN(R)_m[CH_2CH(R')OH]_n \qquad (A)$$

wherein x, y and z are non-zero positive integers the sum of which is from 15 to 21, the sum of y and z being at least 3; m and n are integers the sum of which is 2, n being equal to 1 or 2; R′ represents a hydrogen atom or a methyl group; and R represents an alkyl group.

It will be appreciated that the polyols of formula A contain at least one asymmetric carbon atom and may therefore occur in a plurality of isomeric forms. Each of such isomers, mixtures of such isomers and indeed all mixtures of polyols of formula A are deemed to fall within the scope of the present invention.

Polyols of formula A in which R represents an alkyl group containing 1 to 4 carbon atoms or wherein n is 2 are generally preferred as are polyols of formula A in which R′ represents a hydrogen atom.

It will be apparent that more than one starting material may be utilized to obtain the polyols of the present invention. Conveniently, an unsaturated alcohol, such as oleyl alcohol, may be hydroformylated to give a material which has a terminal hydroxyl functionality from the starting alcohol and a formyl group located toward the center of the molecule. A reductive amination is then conducted on such a material utilizing ammonia or an amine $RNH_2$ and hydrogen to convert the formyl group to a primary or secondary amine structure. Thereafter, primary or secondary amines thus prepared may be converted via an alkylene oxide such as ethylene oxide or propylene oxide to N,N-bis(hydroxyalkyl) or N-hydroxyalkyl substituted amino alcohols.

Thus, starting with oleyl alcohol, one would first obtain 9(10) formyloctadecanol. This material is then converted through the reductive amination to 9(10)-aminomethyloctadecanol. The 9(10)-aminomethyloctadecanol, when reacted with the ethylene oxide, then gives 9(10)-N,N-bis(2-hydroxyethyl)octadecanol. Any unsaturated linear primary alcohol having from 12 to 20 carbon atoms may be utilized to obtain the polyols of the present invention. It is also possible to use polyunsaturated linear primary alcohols, particularly when utilizing cobalt as a catalyst as the hydroformylation reaction has been observed to produce only a monoformyl derivative while reducing any additional unsaturation in the molecule. Thus, linoleyl or linolenyl alcohol yield only monoformyloctadecanol which is then derivatized as discussed above.

Additional starting materials which may be utilized to obtain compounds within the scope of the present invention include unsaturated nitriles such as oleonitrile. In this case, the hydroformylation reaction proceeds with carbon monoxide and hydrogen gas to give 9(10) formyloctadecanonitrile. This formyl compound is then reduced with hydrogen, conveniently using a hydrogenation catalyst, to give 9(10)-hydroxymethyloctadecylamine. This latter amine may then be reacted as described above with ethylene or propylene oxide to give 9(10)-hydroxymethyl-N,N-bis(2-hydroxyalkyl)octadecylamine or may be mono-alkylated to introduce the group R prior to reaction with ethylene or propylene oxide.

The compounds as described above are particularly useful in that a tertiary amine group is present in the molecule which causes the compound to be autocatalytic when utilized in urethane

compositions. That is, the tertiary amine group in the molecule catalyzes the urethane reaction while the hydroxyl group is reactive. Thus, the polyols of the present invention are useful as reactants with polyisocyanates, particularly for reaction injection molding applications where a fast reaction is required. If desired, other polyols may be present in such a reaction. It is also noted that the polyols of the present invention being autocatalytic reduce or eliminate the need for additional catalysts. Such other catalysts are undesired in some particular applications in which the polyols of the present invention are utilized.

According to a further aspect of the present invention we therefore provide a process for the preparation of a polyol of formula A which process comprises reacting an amino-alcohol of formula B

$$CH_3(CH_2)_xCH[(CH_2)_yOH](CH_2)_zNHR \qquad (B)$$

(in which x, y and z are as hereinbefore defined and R represents a hydrogen atom or an alkyl group) with ethylene oxide or propylene oxide.

In the formulae A and B, the sum of the integers x plus y plus z is preferably from 16 to 20, and x, y, and z are each preferably 2, 3, 4 or greater. Among the various preferred polyols of this invention are:

(I) $CH_3(CH_2)_{8(7)}CH[CH_2N(CH_2CH_2OH)_2](CH_2)_{7(8)}CH_2OH$

(II) $CH_3(CH_2)_{8(7)}CH[CH_2N(CH_2CH(CH_3)OH)_2](CH_2)_{7(8)}CH_2OH$

(III) $CH_3(CH_2)_{8(7)}CH(CH_2OH)(CH_2)_{7(8)}CH_2N(CH_2CH_2OH)_2$

(IV) $CH_3(CH_2)_{8(7)}CH(CH_2OH)(CH_2)_{7(8)}CH_2N[CH_2CH(CH_3)OH]_2$

Higher alkylene oxide derivatives may be obtained from the tertiary amino polyols. That is, the alkylene oxide adducts are formed from any of the hydroxyl groups.

According to a yet further aspect of the present invention we provide higher alkylene oxide adducts of polyols of formula A.

According to a yet still further aspect of the present invention we provide a process for the preparation of polyurethanes which process comprises reacting a polyisocyanate with a polyol of formula A or a higher alkylene oxide adduct thereof.

The following Examples are provided to illustrate the preparation of the compounds of the present invention:

Example I: Preparation of formyloctadecanol

The manufacture of formyloctadecanol for use in the preparation of polyols of formula A is accomplished by charging a litre Magne Drive, 316 SS autoclave with 606 grams (2.26 moles) of oleyl alcohol, 3.01 grams of 5% by weight rhodium on alumina and 3 grams (9.68 millimoles) of triphenylphosphite.

The autoclave is sealed and pressurized to 10 atmospheres with nitrogen under stirring and then vented to atmospheric pressure. The nitrogen purge is repeated twice more to ensure removal of any oxygen present in the autoclave.

The autoclave is then pressurized with premixed carbon monoxide and hydrogen gas in a 1:1 molar ratio to 68 atmospheres at which point heating is started. Stirring is manually controlled at 1250 rpm and the uptake of the mixture of the gases starts at about 100 degrees C.

The reaction conditions are then maintained at a temperature of 130 degrees C and a gas pressure of 70 and 75 atmospheres.

The reaction is substantially complete after 4.6 hours and is determined by the cessation of the gas uptake. The confirmation of completeness of the reaction is obtained by sampling the mixture and determining through gas chromatograph analysis that there is less than 1 percent of the starting alcohol in the mixture.

The reaction mixture is then cooled to 75 degrees C, vented to atmospheric pressure and purged twice with nitrogen. The contents of the autoclave are then discharged at 75 degrees C under nitrogen pressure through a pressure filter. The yield of formyloctadecanol is greater than 90 percent.

The reaction may be modified by using triphenylphosphine in place of the triphenylphosphite. Alternatively oleyl alcohol may be substituted by other unsaturated alcohols such as linoleyl or linolenyl alcohol. The reaction temperature may also be lowered, e.g. to 90 degrees C, although the reaction will take a substantially longer period of time to proceed. As a second alternative, the reaction temperature can be raised, e.g. to about 150 degrees C, and the reaction time considerably shortened. However, some decomposition of the end product may occur at the higher temperatures.

In similar fashion, the mixture of carbon monoxide and hydrogen may be varied as previously described and its pressure may also be varied between about 20 and 500 atmospheres. The lower end of the pressure range, of course, slows the reaction rate down while the higher pressure condition increases the reaction rate.

Example II: Preparation of 9(10)-aminomethyloctadecanol

9(10) aminomethyloctadecanol may be prepared utilizing the formyloctadecanol of the foregoing Example.

Into a one litre 316 SS autoclave equipped with a stirrer, thermocouple and an inlet connected to a positive displacement metering pump are charged 150 grams of absolute ethanol and 30 grams of water-wet Raney nickel.

The autoclave is flushed with nitrogen and sealed. Liquid ammonia in the amount of 150 grams is added to the autoclave using a nitrogen head. The autoclave is then heated to 130 degrees C resulting in a pressure of 50 atmospheres absolute. The pressure in the system is increased to 61 atmospheres absolute using hydrogen.

The metering pump is then charged with 301 grams of formyl-octadecanol such as that obtained according to Example 1. The formyloctadecanol is pumped into the autoclave with stirring over a period of 24 minutes during which time the temperature is controlled to the range of about 128 to 132 degrees C and the pressure is controlled at from about 57 to about 61 atmosphere absolute. The reaction process is then maintained under the above conditions for an additional two hours after the addition of the formyloctadecanol is complete. The autoclave is then cooled, vented and the product discharged through a filter using nitrogen pressure. The product is stripped of solvent at about 65 degrees C under a pressure of less than one torr.

The yield of aminomethyloctadecanol is 292 grams having the following analysis. Hydroxyl equivalent weight: 154. Total amine equivalent weight: 352. Secondary plus tertiary amine: 12 meq/kg.

Example III: Preparation of N,N-bis (2-hydroxymethyl)-aminomethyloctadecanol

A 1 litre autoclave equipped with stirrer, ethylene oxide inlet system, sampling tube and thermocouple is first obtained. Into the autoclave is introduced 452 grams of aminomethyloctadecanol such as that obtained according to Example II.

The autoclave is sealed and flushed with nitrogen three times to exclude substantially all oxygen from the reaction mixture. The reaction mass in the autoclave is heated to about 52 degrees C and the ethylene oxide is slowly added over a period of approximately 2 1/2 hours. The temperature is maintained between 52 and 63 degrees C by cooling during the addition of the ethylene oxide. After addition of the ethylene oxide is complete the temperature is maintained at from 54—61 degrees C for an additional 1.5 hours.

The reaction vessel is then cooled to 32 degrees C and allowed to stand for approximately 16 hours. The reaction is then vented to the atmosphere and the product stripped of volatiles under high vacuum at 70 degrees C and less than 1 torr. The yield is observed to be 527 grams having an acetylation equivalent weight of 136 and the total amine equivalent weight of 435. This reaction product corresponds to 9(10)N,N-bis(2-hydroxyethyl)aminomethyloctadecanol.

Shown in Table I below is additional information concerning the preparation of 9(10)N,N-bis(2-hydroxyethyl)aminomethyloctadecanol.

Substantially similar results are obtained when propylene oxide is employed in place of ethylene oxide.

TABLE I

Preparation of N,N-bis(2-hydroxyethyl)aminomethyloctadecanol

| Amino alcohol g (moles) | Ethylene oxide g (moles) | Temp °C | Reaction time (hrs) | Yield (g) | OH eq wt | Total amine eq wt |
|---|---|---|---|---|---|---|
| 267 (0.89) | 104 (2.36) | 39—60 | 10 | 303 | 142 | 462 |
| 454 | 121 (2.75) | 45—55 | 8.0 | 546 | 138 | 460 |
| 452 | 121 | 52—63 | 4.0 | 527 | 136 | 435 |
| 438 | 115 (2.61) | 45—61 | 5.0 | 523 | 134 | 436 |

Example IV: Preparation of 9(10)-hydroxymethyl-N,N-bis(2-hydroxymethylpropyl)-octadecylamine

The compounds of this Example are prepared by obtaining 9(10)-formyloctadecylnitrile which is reduced according to the method of R. Lai (supra) to 9(10) hydroxymethylocta-decylnitrile using hydrogen and a hydrogenation catalyst (e.g. Ni or Co). 9(10)-Hydroxymethyloctadecylamine is then prepared from the nitrile again according to the method of R. Lai.

The 9(10)-hydroxymethyloctadecylamine material is then reacted with two moles of propylene oxide according to a method analogous to that of Example III to obtain 9(10)-hydroxymethyl-N,N-bis(2-hydroxymethylpropyl)octadecylamine. Additional propylene oxide may be used to form higher alkylene oxide condensates.

Example V: Preparation of elastomers

Cast elastomeric products are formulated utilizing N,N-bis(2-hydroxyethyl)aminomethyloctadecanol. Such products are formulated according to Table II shown below.

TABLE II

| Component | Product A* | Product B* |
|---|---|---|
| N,N-bis(2-hydroxyethyl)-aminoethyloctadecanol | 3/11.5 | 3/11.5 |
| Multranol 9151 (polyol from Mobay) | —/— | 0.5/31.5 |
| Niax 31—28 (polyol from Union Carbide | 0.5/31.5 | —/— |
| 1,4-Butanediol | 5.0/7.0 | 5.0/7.0 |
| Mondur PF (polyisocyanate from Mobay) | 8.5/48.0 | 8.5/48.0 |
| Freon 11B (foaming agent) | —/2.0 | —/2.0 |
| Catalyst T-12 (DBTDL) | —/0.005 | —/0.005 |
| Setting Time Min. | 1.0 | 1.5 |
| Demoulding Time Min. | 2.0 | 3.0 |
| Curing Time, 120°C Hr. | 1.0 | 1.0 |

*The first figure in each category is the number of equivalents and the second figure is the percentage by weight in the composition.

The procedure for manufacturing the cast elastomers is as follows:—

The hydroxyl components are combined, demoisturized and degassed at 60 degrees C under a vacuum. The equivalent amount of the isocyanate, the foaming agent and the urethane catalyst are added to the hydroxyl components. The contents of the elastomers formulation are stirred for 20 seconds then poured into a preheated mold and a press having a 4545 kilogram load is applied. The elastomer is allowed to set which occurs in about one minute. The elastomer is then demolded and placed in an oven at 120 degrees C for one hour for final curing.

Product A and Product B are evaluated below in Table III. Product A and Product B may also be used for reaction injection molding (RIM) compositions.

TABLE III

Physical properties of elastomers at —20°F (—28.9°C)

| | Product A | Product B |
|---|---|---|
| Shore A at ambient temp. | 73 | 73 |
| Tensile strength, psi at —20°F (—28.9°C) | 5,500 *($3.792 \times 10^7$) | 11,834 *($8.159 \times 10^7$) |
| Elongation, % at —20°F (—28.9°C) | 0 | 0 |
| Elongation set, % at —20°F (—28.9°C) | 0 | 0 |
| Modulus of elasticity in bending, psi at —20°F (—28.9°C) | 230,790 *($1.591 \times 10^9$) | 326,797 *($2.253 \times 10^9$) |

* Figures in brackets are in $Nm^{-2}$

## Claims

1. A tertiary amino polyol of formula A

$$CH_3(CH_2)_xCH[(CH_2)_yOH](CH_2)_zN(R)_m[CH_2CH(R')OH]_n \quad (A)$$

wherein x, y and z are non-zero positive integers the sum of which is from 15 to 21, the sum of y and z being at least 3; m and n are integers the sum of which is 2, n being equal to 1 or 2; R′ represents a hydrogen atom or a methyl group; and R represents an alkyl group.

2. A polyol as claimed in claim 1 wherein y or z is 1.

3. A polyol as claimed in claim 1 wherein x, y and z each are 2 or greater.

4. A polyol as claimed in any one of claims 1 to 3 wherein the sum of x, y and z is from 16 to 20.

5. A polyol as claimed in any one of claims 1 to 4 wherein R′ represents a hydrogen atom.

6. A polyol as claimed in any one of claims 1 to 5 wherein R represents an alkyl group having 1 to 4 carbon atoms.

7. A polyol as claimed in claim 1 selected from

(a) $CH_3(CH_2)_{8(7)}CH[CH_2N(CH_2CH_2OH)_2](CH_2)_{7(8)}CH_2OH$;

(b) $CH_3(CH_2)_{8(7)}CH[CH_2N(CH_2CH(CH_3)OH)_2](CH_2)_{7(8)}CH_2OH$;

(c) $CH_3(CH_2)_{8(7)}CH(CH_2OH)(CH_2)_{7(8)}CH_2N(CH_2CH_2OH)_2$; and

(d) $CH_3(CH_2)_{8(7)}CH(CH_2OH)(CH_2)_{7(8)}CH_2N[CH_2CH(CH_3)OH]_2$

8. Higher alkylene oxide adducts of polyols of formula A as claimed in any one of claims 1 to 7.

9. A process for the preparation of a polyol of formula A (as defined in claim 1) which process comprises reacting an amino-alcohol of formula B

$$CH_3(CH_2)_xCH[(CH_2)_yOH](CH_2)_zNHR \quad (B)$$

(wherein x, y and z are as defined in claim 1 and R represents a hydrogen atom or an alkyl group) with ethylene oxide or propylene oxide.

10. A process for the preparation of polyurethanes which process comprises reacting a polyisocyanate with a polyol of formula A (as defined in claim 1) or a higher alkylene oxide adduct thereof.

## Patentansprüche

1. Tertiäres Aminopolyol der Formel A

$$CH_3(CH_2)_xCH[(CH_2)_yOH](CH_2)_zN(R)_m[CH_2CH(R')OH]_n \quad (A)$$

in der

x, y und z von Null verschiedene, positive ganze Zahlen sind, deren Summe 15 bis 21 beträgt, wobei die Summe aus y und z wenigstens 3 ist,

m und n ganze Zahlen sind, deren Summe 2 beträgt, wobei n gleich 1 oder 2 ist,

R′ ein Wasserstoff-Atom oder eine Methyl-Gruppe bezeichnet und

R eine Alkyl-Gruppe bezeichnet.

2. Polyol nach Anspruch 1, dadurch gekennzeichnet, daß y oder z 1 ist.

3. Polyol nach Anspruch 1, dadurch gekennzeichnet, daß x, y und z jeweils 2 oder größer sind.

4. Polyol nach irgendeinem der Ansprüche 1 bis 3, dadurch gekennzeichnet, daß die Summe aus x, y und z 16 bis 20 beträgt.

5. Polyol nach irgendeinem der Ansprüche 1 bis 4, dadurch gekennzeichnet, daß R′ ein Wasserstoff-Atome bezeichnet.

6. Polyol nach irgendeinem der Ansprüche 1 bis 5, dadurch gekennzeichnet, daß R eine Alkyl-Gruppe mit 1 bis 4 Kohlenstoff-Atomen bezeichnet.

7. Polyol nach Anspruch 1, ausgewählt aus

(a) $CH_3(CH_2)_{8(7)}CH[CH_2N(CH_2CH_2OH)_2](CH_2)_{7(8)}CH_2OH$;

(b) $CH_3(CH_2)_{8(7)}CH[CH_2N(CH_2CH(CH_3)OH)_2](CH_2)_{7(8)}CH_2OH$;

(c) $CH_3(CH_2)_{8(7)}CH(CH_2OH)(CH_2)_{7(8)}CH_2N(CH_2CH_2OH)_2$ und

(d) $CH_3(CH_2)_{8(7)}CH(CH_2OH)(CH_2)_{7(8)}CH_2N[CH_2CH(CH_3)OH]_2$.

8. Höhere Alkylenoxid-Addukte der Polyole der Formel A nach irgendeinem der Ansprüche 1 bis 7.

9. Verfahren zur Herstellung eines Polyols der Formel A (nach Anspruch 1), dadurch gekennzeichnet, daß ein Aminoalkohol der Formel B

$$CH_3(CH_2)_xCH[(CH_2)_yOH](CH_2)_zNHR \qquad (B)$$

(in der x, y und z die in Anspruch 1 angegebenen Bedeutungen haben und R ein Wasserstoff-Atom oder eine Alkyl-Gruppe bezeichnet) mit Ethylenoxid oder Propylenoxid umgesetzt wird.

10. Verfahren zur Herstellung von Polyurethanen, dadurch gekennzeichnet, daß ein Polyisocyanat mit einem Polyol der Formel A (nach Anspruch 1) oder einem höheren Alkylenoxid-Addukt desselben umgesetzt wird.

**Revendications**

1. Un amino polyol tertiaire de formule:

$$CH_3(CH_2)_xCH[(CH_2)_yOH](CH_2)_zN(R)_m[CH_2CH(R')OH]_n \qquad (A)$$

dans laquelle x, y et z sont des nombres entiers positifs différents de 0, dont la somme est de 15 à 21, la somme de y et de z étant d'au moins 3; m et n sont des nombres entiers dont la somme est 2, n étant égal à 1 ou 2; R' représente un atome d'hydrogène ou un groupe méthyle; et R représente un groupe alkyle.

2. Un polyol tel que revendiqué dans la revendication 1, dans lequel y ou z est 1.

3. Un polyol tel que revendiqué dans la revendication 1, dans lequel x, y et z représentent chacun 2 ou un nombre entier supérieur.

4. Un polyol tel que revendiqué dans l'une quelconque des revendications 1 à 3, dans lequel la somme de x, y et z est de 16 à 20.

5. Un polyol tel que revendiqué dans l'une quelconque des revendications 1 à 4, dans lequel R' représente un atome d'hydrogène.

6. Un polyol tel que revendiqué dans l'une quelconque des revendications 1 à 5, dans lequel R représente un groupe alkyle ayant de 1 à 4 atomes de carbone.

7. Un polyol tel que revendiqué dans la revendication 1, choisi parmi:

(a) $CH_3(CH_2)_{8(7)}CH[CH_2N(CH_2CH_2OH)_2](CH_2)_{7(8)}CH_2OH$;

(b) $CH_3(CH_2)_{8(7)}CH[CH_2N(CH_2CH(CH_3)OH)_2](CH_2)_{7(8)}CH_2OH$;

(c) $CH_3(CH_2)_{8(7)}CH(CH_2OH)(CH_2)_{7(8)}CH_2N(CH_2CH_2OH)_2$, et

(d) $CH_3(CH_2)_{8(7)}CH(CH_2OH)(CH_2)_{7(8)}CH_2N[CH_2CH(CH_3)OH]_2$

8. Produits d'addition supérieurs d'oxyde d'alkylène et de polyols de formule A tels que revendiqués dans l'une quelconque des revendications 1 à 7.

9. Un procédé pour la préparation d'un polyol de formule A (tel que défini dans la revendication 1) lequel procédé consiste à faire réagir un amino-alcool de formule B

$$CH_3(CH_2)_xCH[(CH_2)_yOH](CH_2)_zNHR \qquad (B)$$

(dans laquelle x, y et z sont tels que définis dans la revendication 1 et R représente un atome d'hydrogène ou un groupe alkyle) avec de l'oxyde d'éthylène ou de l'oxyde de propylène.

10. Un procédé pour la préparation de polyuréthanes, ce procédé consistant à faire réagir un polyisocyanate avec un polyol de formule A (tel que défini dans la revendication 1) ou un de ses produits d'addition supérieurs d'oxyde d'alkylène.